# EUROPEAN PATENT APPLICATION

(11) **EP 4 717 190 A1**
(43) Date of publication of application: **01.04.2026**
(21) Application number: 24203260.5
(22) Date of filing: 27.09.2024
(51) Int. Cl.: A61B 17/02, A61F 9/00

(54) **EYE GRIPPING DEVICE AND EYE MANIPULATION SYSTEM**

(71) Applicant: Carl Zeiss Meditec AG, 07745 Jena (DE)
(72) Inventor: MEENINK, Hildebert Christiaan Matthijs, 5656 AG Eindhoven (NL); LINSSEN, Albertus Joannes Maria, 5656 AG Eindhoven (NL)
(74) Representative: DeltaPatents B.V.

(57) **Abstract**

An eye gripping device 120 is provided for engaging an eyeball 102. The eye gripping device comprises a base structure that includes one or more contact elements 126. The contact elements define one or more contact surfaces arranged along a contact plane that conforms to the curvature of the ocular surface. The device further includes a plurality of needles 128 affixed to the base structure, each needle having an outer diameter of 600 µm or less, preferably 400 µm or less, and more preferably 200 µm or less. The needles protrude from the contact plane by 600 µm or less, preferably 400 µm or less, facilitating secure engagement with the ocular surface without penetrating deeply into the tissue. The device is suitable for precise eye manipulation, ensuring stability during procedures involving the eyeball 102.

## Description

### TECHNICAL FIELD

The presently disclosed subject matter relates to an eye gripping device for engaging an eyeball, and to an eye manipulation system for orbital manipulation of an eye of a patient, the eye manipulation system comprising the eye gripping device.

### BACKGROUND

Ophthalmic surgical procedures increasingly involve the use of surgical robotic systems. These systems offer numerous benefits, including enhanced precision and dexterity. However, rather than operating entirely autonomously, such surgical robotic systems are expected to remain at least partially under human operator control for the near future. For example, the human operator may directly or indirectly control the movement of a surgical instrument mounted on a surgical arm of the robotic system. Nevertheless, it is anticipated that future surgical robotic systems may become more autonomous, eventually requiring less or even no human involvement.

While the use of surgical robotic systems in ophthalmic procedures offers numerous benefits, including enhanced precision and dexterity, it is considered crucial that the eye's pose relative to the surgical instrument is known during surgery to ensure appropriate control of the instrument. Here, the 'pose of the eye' includes both its position and orientation of the eye. The eye's position may be determined by or based on the patient's head position, while its orientation may be determined by both the head orientation and the eye's orientation within the socket. For example, during intraocular surgery, the patient's head may be brought into a known position and orientation, and the eye's orientation within the socket may be fixed. One known method is to establish a coupling between the surgical robotic system and the eye using a trocar interface, thereby fixing the eye's orientation relative to the robotic system.

US2021000566A1 describes a docking system for intraocular surgery that includes a rigid support structure. This structure has a first end that accommodates, receives, and fits over an imaging probe, and a second end that accommodates and receives the eyeball to secure it relative to the imaging probe. The system incorporates a retaining mechanism, such as a passive suction attachment, at or adjacent to the second end of the support structure to secure the eyeball. This suction attachment may be generally circular, such as a ring, and may cover an anterior segment of the eyeball (cornea/limbus/sclera).

A potential drawback of suction-based eye gripping devices such as US2021000566A1's docking system is that these devices are generally applied to the cornea since the cornea has a sufficiently hard surface to ensure adequate grip. However, a corneal application has clear disadvantages since it typically obstructs the surgeon's view and impedes instrument access during ophthalmic procedures.

Another potential drawback of US2021000566A1 is maintaining optimal suction levels. Sufficient suction is needed to secure the eye, but excessive suction may risk increasing intraocular pressure, potentially affecting delicate ocular structures or compromising surgical precision. In an attempt to address this, US2021000566A1 incorporates a pressure sensor and regulation valve to monitor and control internal pressure. However, this need for continuous suction management adds complexity to the surgical process, potentially requiring additional equipment and attention from the surgical team to ensure patient safety and optimal conditions.

It would be desirable to provide an eye gripping device for engaging an eyeball that can also be applied to other areas of the eye, such as the sclera, and that that is less complex to use during an ophthalmic surgical procedure.

In addition to the static fixation of the eye, as described in US2021000566A1, there may also be a need to manipulate the eye's orientation during an ophthalmic surgical procedure. Such manipulation may involve rotating the eye within its socket, thereby changing its orientation with respect to one or more external surgical entities, such as the instrument manipulator of a surgical robotic system or a microscope. This externally induced change in the eye's orientation may also be referred to as orbital manipulation, as it involves altering the eye's orientation within the orbital cavity. Such manipulation may be advantageous for various reasons, including but not limited to improving the visualization or observability of peripheral regions of the eye and enhancing the reach of surgical instruments within the eye. It would be desirable for an eye gripping device to also be suitable for orbital manipulation

### SUMMARY

In accordance with a first aspect of the presently disclosed subject matter, an eye gripping device is provided for engaging an eyeball, comprising:
- a base structure comprising one or more contact elements, wherein the one or more contact elements define one or more contact surfaces for contacting an ocular surface of the eyeball, wherein the one or more contact surfaces are arranged along a contact plane that conforms to a curvature of the ocular surface;
- a plurality of needles affixed to the base structure, wherein respective needles have an outer diameter of 600 µm or less, preferably 400 µm or less, more preferably 200 µm or less, and protrude by 600 µm or less, preferably 400 µm or less, from the contact plane.

The eye gripping device as described above is designed to engage the eyeball using a plurality of needles that pierce the ocular surface when the eye gripping device is applied to the eyeball. To minimize trauma to the eye, individual needles have specific dimensions, namely an outer diameter of 600 µm or less, preferably 400 µm or less, and more preferably 200 µm or less. Such small outer diameters typically allow for healing of the ocular surface of the eye without the need for sutures, which may in turn allow faster recovery of the patient and reduce the risk of infection.

The needles, which may also be referred to as micro-needles due to their small outer diameter, may be designed to pierce a fibrous surface layer of the eyeball, which allows the eye gripping device to exert a grip on the eyeball. The fibrous surface layer may include structures such as the sclera and the cornea, which together form a protective outer coating for the eye. To avoid completely penetrating through this fibrous surface layer, and thereby compromising the protective outer coating of the eye, individual needles may protrude by 600 µm or less, and preferably 400 µm or less, from the contact plane of the eye gripping device. By limiting the protrusion depth, it may be avoided that the needles penetrate through the protective outer coating of the eye. On the other hand, to ensure sufficient penetration through for example the loose conjunctiva and into the fibrous surface layer of the sclera, individual needles may protrude at least 100 µm or more, preferably 200 µm or more, from the contact plane. This length may allow the needles to fully penetrate through the bulbar conjunctiva which covers the white part of the eye and has a thickness of approximately 33 µm.

The contact plane may be further explained as follows. The eye gripping device includes a base structure which comprises, or is comprised of, one or more contact elements. Each contact element may have a surface that is intended to contact the ocular surface of the eyeball. The surfaces, also called contact surfaces or engaging surfaces, may be arranged to lie along a plane that substantially conforms to the ocular surface's curvature. This plane is elsewhere also referred to as a contact plane. In other words, the structural and geometric arrangement of the contact elements may be designed such that the contact surfaces contact the ocular surface when the eye gripping device is applied to the eyeball, allowing the eye gripping device to conform to the curved shape of the eyeball. The curvature which the eye gripping device may be designed to conform to may be a physiological average curvature of the eye. It may thus be known beforehand which part(s) of the eye gripping device will or are likely to contact the ocular surface. This may enable precise control over the insertion depth of the needles since needles which protrude beyond the contact plane will likely penetrate the ocular surface. Accordingly, the needles may be affixed to the base structure such that their protrusion from the contact plane is limited to avoid completely penetrating the fibrous layer of the eyeball, e.g., by protruding 600 µm or less, preferably 400 µm or less from the contact plane. Generally, the protrusion length of the needles from the contact plane may be selected to allow needles to penetrate into the fibrous layer up to, but not penetrating, the underlying choroidal layer.

Unlike suction-only devices that typically require corneal application for sufficient grip, the eye gripping device described above can achieve adequate fixation when applied to other areas, such as the sclera. This versatility allows for designs that maintain access to the cornea, preserving line-of-sight for microscopes, enabling surgical instrument access, and facilitating corneal moisturization during procedures. In contrast, suction-based devices often necessitate gripping a large portion of the cornea to exert sufficient grip for orbital manipulation, inherently limiting such access.

When compared to suction-only based devices, the eye gripping device as described above may not require continuous and accurate control of suction levels and thereby avoid the complexities associated with such control. Moreover, suction-only based devices may require airtight edges, often achieved with small contact surfaces that can cause high local stresses and potentially deform the eye. The eye gripping device may not require such small contact surfaces, reducing the risk of damage.

Optionally, the base structure comprises a central section which comprises an aperture to expose at least part of the cornea when the eye gripping device engages the eyeball. By providing a central section which comprises an explicitly defined aperture, e.g., an opening, access to the cornea is largely maintained when the eye gripping device engages the eye. It is therefore avoided that the eye gripping device completely or substantially obstructs access to the cornea and the eye's interior.

Optionally, the one or more contact elements are arranged radially around the central section to contact respective parts of the sclera when the eye gripping device engages the eyeball. By radially arranging the contact elements around the central section, the force exerted by the needles, e.g., when using the eye gripping device for orbital manipulation, may be distributed radially around the cornea. This distribution may be advantageous since it may help to reduce or minimize localized pressure on the eye, reducing the risk of corneal deformation or damage.

Optionally, the one or more contact elements form a ring-shaped structure or one or more of radial segments of a ring-shaped structure, wherein the ring-shaped structure has an inner diameter of at least 8 mm, preferably at least 10 mm, more preferably 12 mm. A ring-shaped structure may allow the forces exerted by the needles to be distributed evenly or substantially evenly around the cornea, while at the same time continuing to provide access to the cornea for surgical instruments.

Optionally, the plurality of needles protrude from the one or more contact surfaces of the one or more contact elements. When the needles protrude from the contact surfaces of the contact elements, the penetration depth may be precisely controlled. Specifically, the degree of protrusion, for example measured in millimeters, may directly correspond to the depth of penetration into the fibrous surface layer. Advantageously, the risk of excessive needle penetration may be easily mitigated, as the contact surface, when the eye gripping device engages the eye, rests on the ocular surface. This positioning inherently limits the needles from penetrating too deeply.

Optionally, the plurality of needles are arranged as respective needle beds on the one or more contact surfaces. Here, the term 'needle bed' may refer to a configuration where multiple needles are grouped closely together in a defined pattern on the contact surface. Such needle beds may be well suited for distributing the forces exerted by individual needles across a larger surface area, thereby helping to minimize localized pressure on the eye and reducing the risk of tissue damage or deformation. For example, the density of needles in a needle bed may be selected proportionally to the outer diameter of the needles. For example, the pitch between needles in a needle bed may be selected between 2 and 8 times the outer diameter, preferably between 3 and 6 times the outer diameter, more preferably between 3.5 and 4.5 times the outer diameter. In a specific example, the pitch may be approximately 4x the outer diameter.

Optionally, a protrusion depth of a respective needle is selected to remain below an average physiological thickness of the sclera at a respective position of the needle. By ensuring that the protrusion depth of individual needles remains below this average thickness, it may be avoided that the needles completely penetrate through the fibrous layer of the eyeball, thus preserving the integrity of the eye's protective outer coating. Since the average physiological thickness of the sclera varies across the eye's surface, the penetration depth of individual needles may be adjusted accordingly, taking into account the intended placement of the eye gripping device. Optionally, the protrusion depth may be set to remain below the overall average physiological thickness of the sclera. Additionally, the protrusion depth may be configured to remain below the typically minimum physiological thickness of the sclera at the intended placement of the eye gripping device, thereby reducing the risk of penetration regardless of the exact placement of the eye gripping device. For example, the protrusion depth may remain below the aforementioned 600 µm or less, preferably 400 µm or less, and preferably 300 µm or less for needles to be placed near the limbus.

Optionally, the plurality of needles protrude by different lengths from the contact plane, wherein needles which are affixed more centrally to the base structure protrude less from the contact plane than needles which are affixed more peripherally to the base structure. Since the sclera is on average thinner near the cornea, e.g., reaching a minimum thickness of around 300 µm near the limbus where the cornea meets the sclera, the protrusion depth of needles intended to penetrate closer to the cornea may be selected to be less than the penetration depth of needles positioned further from the cornea. This approach may achieve a balance between safety, namely by reducing the risk of puncturing the eye's protective outer layer, and effective grip by ensuring that penetration is not too shallow where greater depth is permissible.

Optionally, the plurality of needles comprise needles which are oriented at different angles with respect to the contact plane. The use of needles which penetrate the fibrous surface layer at different angles may allow for a firmer grip of the eyeball.

Optionally, the eye gripping device further comprises an adjustment mechanism to adjust a protrusion depth of the plurality of needles. The ability to adjust the protrusion depth of the needles may be advantageous in accommodating patient variability or variations in device placement. Moreover, it may allow for the needles to be gradually protruded, for example, when the eye gripping device is already positioned on the ocular surface. Such gradual protrusion may have the advantage of minimizing sudden pressure on the eye, reducing the risk of tissue damage, and allowing for a more controlled and precise engagement with the ocular surface.

Optionally, the plurality of needles is slidingly affixed to the base structure, wherein the adjustment mechanism comprises a driving element arranged to drive the needles from the base structure to further protrude from an eye-facing side of the base structure. The needles may be slidingly arranged, meaning they may be capable of sliding along their longitudinal direction relative to the base structure. The driving element, which may, for example, be a mechanical component, may be used to extend the needles from the eye-facing side of the base structure. This may allow for precise control over the protrusion depth of the needles, enabling adjustments as needed.

Optionally, the base structure comprises a threaded portion and the driving element is configured to be screwed onto the threaded portion, wherein the driving element is configured to contact proximal ends of respective needles to drive the needles from the base structure when the driving element is screwed onto the threaded portion. The driving element may be operated in a screw-like manner, where the driving element is screwed onto or into a threaded portion of the base structure. As the driving element is screwed onto or into the base structure, the distance between the driving element and the base structure decreases, causing it to engage with the proximal ends of the needles. This screwing motion may gradually drive the needles out of the base structure on the eye-facing side. This mechanism is advantageous as it can be easily manually actuated, for example, by hand, providing precise control over the protrusion depth even after the eye gripping device has been applied to the eye.

Optionally, the adjustment mechanism is arranged to sequentially adjust the protrusion depth of individual needles or individual subsets of needles. This sequential adjustment allows each needle or subset of needles to extend one after the other from the base structure, facilitating a more controlled protrusion into the ocular surface. By avoiding the simultaneous application of all needles, the so-called needle bed effect, where the needles fail to penetrate a surface due to the distributed pressure across multiple points, is mitigated, ensuring effective penetration into the ocular surface.

Optionally, the adjustment mechanism is arranged to sequentially adjust the protrusion depth of the individual needles or individual subset of needles by sequentially driving the individual needles or individual subset of needles with the driving element from the base structure. For example, the driving element may be wedge-shaped and move over the non-eye-facing side of the base structure, pushing the needles at their distal ends to further extend them from the eye-facing side. If combined with a screw-like mechanism, the driving element may rotate, progressively advancing over each needle or group of needles to incrementally push them forward.

Optionally, respective needles comprise a beveled tip. A beveled tip may facilitate easier penetration into the ocular surface. Preferably, the tip is beveled and sharpened to include a defined cutting edge. This cutting edge may be designed to slice through the tissue rather than simply pushing through it, which reduces trauma to the ocular surface. By creating a cut, the beveled, sharpened tip minimizes tissue displacement and promotes faster healing, as the slit can close on its own more effectively compared to the irregular opening created by a blunt or non-sharpened tip.

Optionally, the eye gripping device is configured to exert the grip on the fibrous surface layer of the eyeball at multiple positions or across an area of the surface of the eyeball. By exerting the grip at multiple positions or across an area of the surface of the eyeball, the forces exerted by the eye gripping device may be spatially distributed over at least part of the fibrous surface layer, which may increase the efficacy of the exerted grip and may distribute stress across the fibrous surface layer, which in turn may reduce the localization of potentially damaging stress.

Optionally, the eye gripping device is configured to apply suction to the eye to temporarily stabilize the eye gripping device relative to the eye. This suction may temporarily secure the eye gripping device in place, which may allow the needles to penetrate the ocular surface more effectively and accurately, reducing or minimizing the risk of movement that could lead to imprecise needle placement or potential damage to the eye. For example, the eye gripping device may first be secured to the eye via suction, after which the needles may be extended from the eye gripping device as described previously. To apply suction, the eye gripping device may for example comprise a suction cup or chamber with a controllable vacuum mechanism that can be activated to create a seal between the eye gripping device and the ocular surface.

A further aspect of the presently disclosed subject matter provides an eye manipulation system for orbital manipulation of an eye of a patient, wherein the eye manipulation system comprises the eye gripping device. The eye manipulation system may for example comprise an eye gripping device mounted to an arm. The arm may be a mechanical arm, meaning that the arm may be actuated, for example by hand or directly by one or more actuators or by one or more actuators acting upon a component which moveably suspends the arm. The arm may have at least one degree of freedom which may allow the arm to assume different poses in accordance with the at least one degree of freedom. The eye gripping device may for example be mounted to a distal end of the arm. The term 'distal' may use a base of the arm as a point of reference, meaning that a distal section or end of the arm may be located away from the base and is typically nearest to the surgical target. The at least one degree of freedom of the arm may, mechanically and/or through actuator control, cause the arm to move the eye gripping device along a curved plane representing a curvature of the eyeball. The movement may involve both repositioning and reorienting the eye gripping device, which may allow the eye gripping device to follow the contour of the curved plane in both location and orientation. Through this movement along the contour of the curved plane, the eye gripping device may be guided along a surface of the eye. Since the eye gripping device exerts grip onto the eyeball, the movement of the eye gripping device along the surface of the eye will cause the eyeball to be rotated in its eye socket.

It will be appreciated by those skilled in the art that two or more of the above-mentioned embodiments, implementations, and/or aspects of the presently disclosed subject matter may be combined in any way deemed useful.

Modifications and variations of the eye manipulation system or the surgical robotic system, which correspond to the described modifications, variations, and optional aspects of another one of these entities and activities, may be carried out by a person skilled in the art on the basis of the present description.

### BRIEF DESCRIPTION OF THE DRAWINGS

These and other aspects of the presently disclosed subject matter are apparent from and will be elucidated with reference to the embodiments described hereinafter. In the drawings,
Fig. 1A schematically shows an eye gripping device which comprises a base structure and a plurality of needles affixed to the base structure, wherein the eye gripping device is applied to an eyeball causing the needles to pierce the sclera;
Fig. 2A schematically shows the eye gripping device mounted to an arm;
Fig. 2B schematically shows an actuation of the arm to rotate the eyeball within the eye socket;
Figs. 3A and 3B show part of a base structure of the eye gripping device, showing a contact element in form of a convex plate from which the needles protrude;
Figs. 4A and 4B show part of a base structure of the eye gripping device, showing a convex plate which comprises feet as contact elements, wherein the needles protrude from the convex plate past the contact plane defined by the feet;
Figs. 5A and 5B show part of a base structure of the eye gripping device, showing a contact element in form of a convex plate in which the needles are slidingly arranged, wherein the needles are urged through the convex plate into the sclera by a driving element which pushes the needles in a sequential manner at their proximal end;
Figs. 6A-6D show different arrangements of needles.

It should be noted that items which have the same reference numbers in different figures, have the same structural features and the same functions, or are the same signals. Where the function and/or structure of such an item has been explained, there is no necessity for repeated explanation thereof in the detailed description.

### List of reference numerals

The following list of references and abbreviations is provided for facilitating the interpretation of the drawings and shall not be construed as limiting the claims.
- 102: eyeball
- 104: eye socket
- 106: cornea
- 107: sclera
- 110: surgical instrument

- 120: eye gripping device
- 122: central section
- 124: aperture
- 126: contact element
- 128: needle

- 150: eye manipulation system
- 152: arm
- 154: base
- 160: movement
- 162: rotation

- 200, 202: contact element
- 210, 212: contact plane
- 220, 222: penetration depth
- 230: needle
- 240: driving element

- 300-306: contact element
- 310-316: needle

### DESCRIPTION OF EMBODIMENTS

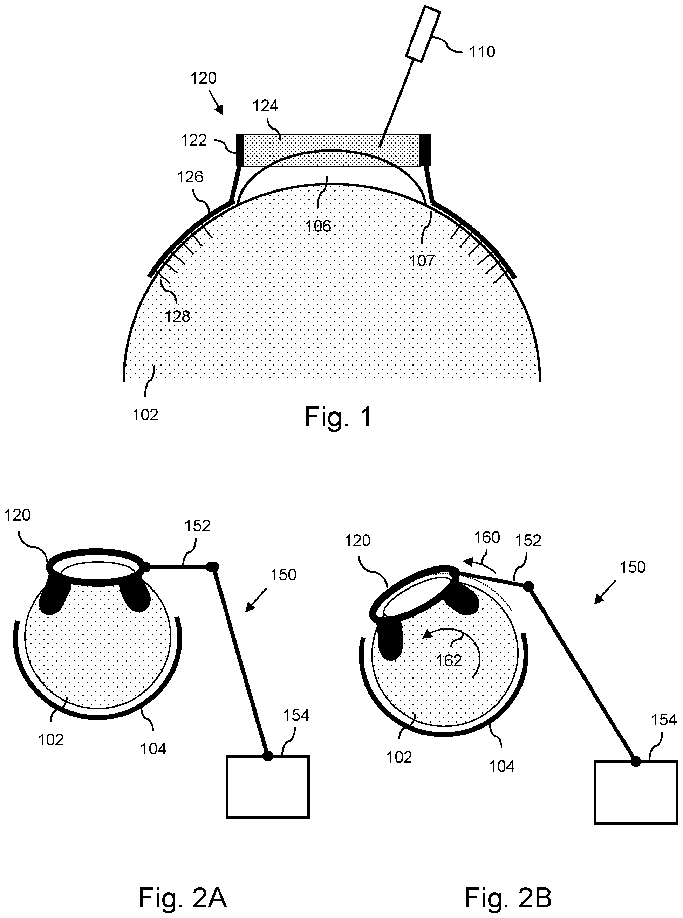

**Fig. 1** illustrates various structures of the eye, including the eyeball 102, the corneal surface layer 106 (also referred to simply as the "cornea"), and the scleral surface layer 107 (also referred to simply as the "sclera"). Other structures of the eye, such as the lens and the eye socket, are not shown.

Fig. 1 further depicts an eye gripping device 120 which is designed to engage the eyeball 102. The eye gripping device 120 is shown to comprise contact elements 126 in the form of convex plates. The term "convex plate" may refer to a flat, plate-like part that is curved. The curvature radius of the convex plates 126 may be designed to substantially match that of the eyeball, e.g., of a physiologically typical eyeball. The convex plates 126 are connected to a central section 122 which includes an aperture 124 that exposes at least part of the cornea 106, allowing access to the cornea, for example for surgical instruments 110 or to moisturize the cornea, or optical access, for example via a surgical microscope. In one possible design, the central section 122 may be ring-shaped, with the interior of the ring providing substantially unobstructed access to the cornea, while the convex plates 126 may be affixed circumferentially around the central section. The inner diameter of the ring may for example be 6, 8, 9, 10, 11, 11.5, 12 mm, etc. Together, the central section 122 and the convex plates 126 may form the base structure of the eye gripping device 120 of Fig. 1.

In addition to the base structure, the eye gripping device 120 may include a number of needles 128 which are affixed to the base structure. Fig. 1 and subsequent figures illustrate a limited number of needles. However, as explained elsewhere, the actual number of needles may vary and may be greater or fewer than shown.

Fig. 1 shows the eye gripping device 120 in an engaged state, with the needles 128 inserted into the eyeball. The penetration depth of the needles 128 is designed to be 600 µm or less, with a preferred depth of 400 µm or less. It should be noted that the protrusion depth shown in Fig. 1 and other figures is not shown to scale. The needles 128 of the eye gripping device 120 of Fig. 1 are generally arranged to penetrate the sclera when the eye gripping device is applied to the eye.

To ensure the correct application of the eye gripping device, a guidance member may be integrated into the design of the eye gripping device 120. This guidance member may assist surgical staff or a robotic system in applying the eye gripping device accurately. For example, the central section 122 may function as the guidance member to ensure that the eye gripping device 120 is placed centrally with respect to the cornea. Specifically, the aperture 124 of the central section 122 may function as guidance member as the eye gripping device may be required to be placed with the aperture centrally over the cornea to ensure that the needles are positioned to correctly puncture the sclera. Alternatively, other types of guidance members may be provided depending on the specific design and application requirements.

Fig. 2A shows an eye manipulation system 150 for orbital manipulation of an eye of a patient. The eye manipulation system 150 is shown to comprise the eye gripping device 120 of Fig. 1, an arm 150, and a base 154. The eye gripping device 120 is shown to be mounted to a distal end of the arm 152. The arm 152 may have at least one degree of freedom and may be connected with its proximate end to the base 154. The arm 152 may be adjustable in pose to move the eye gripping device 120 along a curved plane representing a curvature of the eyeball 102. **Fig 2B** shows a result of such an adjustment in pose, which may for example be effected by actuating the arm 152, for example manually or using one or more actuators, to effect the movement of the eye gripping device 120 along the curved plane. Namely, by way of the movement 160 of the eye gripping device 120, the eyeball 102 may be rotated 162 within the eye socket 104. Such externally and purposefully induced rotation of the eyeball 102 within the eye socket 104 may also be referred to as orbital manipulation. It is noted that actuation of the arm 152 may take place by one or more actuators directly acting upon the arm 152 and/or by one or more actuators acting upon the base 154.

With continued reference to the eye gripping device, the following describes a number of arrangements of contact elements of an eye gripping device.

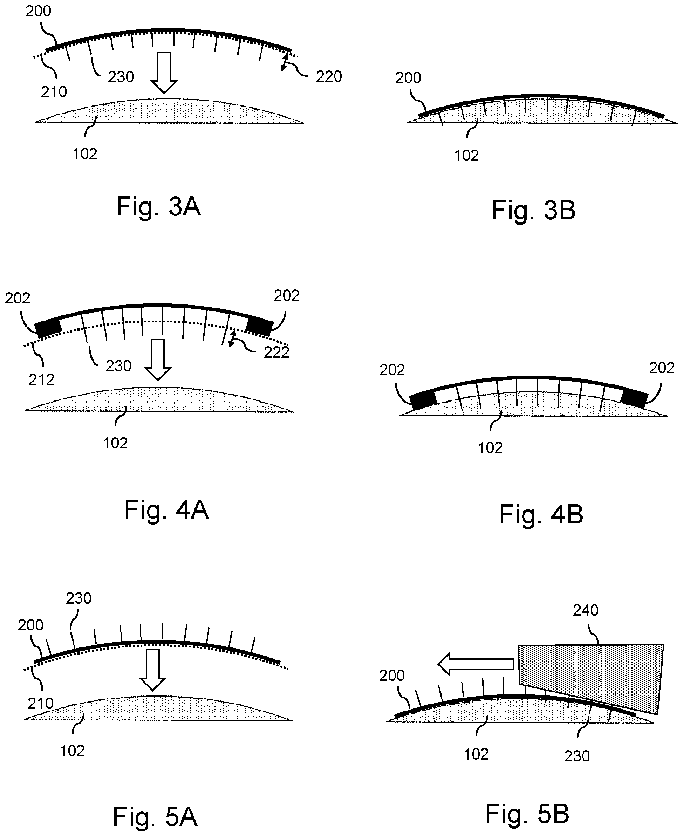

**Figs. 3A and 3B** illustrate a part of an exemplary base structure of the eye gripping device, depicting a contact element in the form of a convex plate 200 from which the needles 230 protrude. In this example, the needles 230 may be affixed to the convex plate 200 in a fixed, non-sliding manner. For example, the needles 230 may be glued or mechanically fastened to the convex plate 200. Another example of affixing the needles 230 to the convex plate 200 is by embedding the base of the needles 230 into the material of the convex plate 200 during a molding or manufacturing process.

As shown in Fig. 3B, the convex plate 200 may come into contact with the ocular surface of the eyeball 102 when the eye gripping device is applied to the eye. To facilitate proper contact across the surface of the convex plate 200, the curvature of the convex plate may be selected to correspond to the typical physiological curvature of the eyeball. For example, the curvature of the convex plate 200 may have a radius of curvature in the range of 11 to 13 mm to match the average curvature of the human eyeball. The inner side of the convex plate 200 thus represents a contact surface with respect to the eyeball 102. A contact plane 210 may be drawn through this contact surface, representing the plane at which contact will be established with the eye. The length of the protrusion of the needles 230 from this contact surface or contact plane 210 may substantially determine the penetration depth 220 into the eyeball 102.

During the design or manufacture of the eye gripping device, the penetration depth 220 may be appropriately selected, for example, to remain below an upper limit of 600 µm, 400 µm, or 300 µm. The penetration depth 220 may be chosen based on the intended contact area between the convex plate 200 and the eyeball 102. For example, closer to the cornea, such as at or near the limbus, the penetration depth 220 may be selected to be shorter, e.g., 300 µm or below, whereas further away from the cornea, a greater upper limit may be selected, e.g., 400 µm or below.

Although not explicitly shown in Figs. 3A-3B, the needles 230 may be affixed to the convex plate 200 to form a needle bed. A needle bed may for example comprise fewer than 10 needles, between 10 and 50 needles, between 50 and 100 needles, between 100 and 250 needles, or more than 250 needles. The density of the needles in a needle bed may be selected proportionally to the outer diameter of the needles. For example, the pitch between the needles may be selected to be between 2 and 8 times the outer diameter of the needles, preferably between 3 and 6 times the outer diameter, and more preferably between 3.5 and 4.5 times the outer diameter. In a specific example, the pitch may be approximately 4 times the outer diameter.

**Figs. 4A and 4B** illustrate a part of an exemplary base structure of the eye gripping device, depicting a convex plate that comprises feet 202 acting as contact elements. In this configuration, the contact surface area is smaller than in the example shown in Figs. 3A-3B. Unlike the previous example, the needles 230 do not directly protrude from the contact surface but instead extend from another part of the base structure, such as a recessed section, which is not in direct contact with the eye.

In this case, the correct penetration depth 222 may be determined by considering the distance the needles 230 protrude beyond the contact plane 212 established by the feet 202. Since the needles 230 originate from a recessed part of the base structure, the protrusion depth 222 should be calculated from this contact plane 212 to ensure proper penetration into the eyeball without exceeding safe limits.

**Figs. 5A and 5B** illustrate a part of an exemplary base structure of the eye gripping device, depicting a contact element in the form of a convex plate 200 in which the needles 230 are slidingly arranged, for example individually, allowing a respective needle to slide independently from another needle, or in groups, allowing a respective group of needles to slide independently from another group. Such groups are elsewhere also referred to as 'subsets'. The needles 230 are urged through the convex plate 200 into the sclera by a driving element 240, which sequentially pushes the needles at their proximal ends. The driving element 240 may be part of an adjustment mechanism designed to adjust the protrusion depth 220 of the plurality of needles 230. The needles 230 may be slidingly affixed to the base structure in various ways, for example, by guide channels, grooves, or a similar mechanism that allows for controlled movement. The adjustment mechanism may include the aforementioned driving element 240, which is arranged to drive the needles 230 from the base structure, causing them to further protrude from the eye-facing side of the convex plate 200.

In the example shown in Figs. 5A-5B, the adjustment mechanism is configured to sequentially adjust the protrusion depth 220 of individual needles 230 or specific subsets of needles. This sequential adjustment is achieved by driving each needle or subset of needles one after each other using the driving element 240. However, this example does not limit the invention, as the protrusion depth 220 of all needles 230 may also be adjusted simultaneously. The sequential driving of the needles 230 may be achieved through various mechanisms. For example, although not explicitly shown in Figs. 5A-5B, the base structure may include a threaded portion, and the driving element 240 may be configured to be screwed onto this threaded portion. In this configuration, the driving element 240 may contact the proximal ends of the needles 230, driving them out from the base structure as it is screwed onto the threaded portion. Another example is that the driving element 240 may be configured with a ratcheting mechanism that incrementally advances the needles 230 in a controlled manner. In this configuration, each activation of the ratchet may cause the driving element 240 to push one or more needles 230 forward by a predetermined amount. The ratcheting mechanism may for example be manually actuated by turning a dial or lever, or the mechanism may be motorized for automated adjustment. The ratcheting mechanism may additionally be configured to incrementally retract the needles 230 in a controlled manner, for example by the ratcheting mechanism being operated in reverse.

In general, the driving element 240 may have a slanted surface, for example by the driving element 240 having a wedge or wedge-like shape, which, when moved over the proximal ends of the needles 230, incrementally drives the needles out from the base structure. Here, 'incrementally' may refer to needles being driven out one-by-one or one group by another group. Another example is that the driving element may have a wheel-like or cylindrical shape which enables the driving element to roll over the proximal ends of the needles and thereby apply a force onto the proximal ends of the needles. The driving element 240 may be actuated manually, such as by hand, or through other means, such as a motorized mechanism or pneumatic actuator, depending on the specific design and application of the eye gripping device.

In addition to driving the needles out from the base structure, the needles may also be retractable, for example using a separate retraction mechanism or by the driving element being additionally configured to retract the needles. One example is that the needles may be spring-loaded within the base structure so that they naturally maintain a retracted position. The driving element may then extend the needles at their distal ends in a controlled manner from the base structure by exerting force on their proximal ends, and cause the needles to retract into the base structure by removing the force, either all at once or incrementally. Another example is that the needles may be connected to a rotating cam which may, when rotated, cyclically extend, and retract the needles. Yet another example is that needles may be retracted magnetically. Another example is that the needles may be equipped with small hooks or flanges at their proximal ends, allowing a mechanical gripper to engage and retract the needle. Yet another example is that the needles may be arranged on a flexible lint, belt, or chain, which may allow the needles to be retracted by retracting the lint, belt, or chain.

While Figs. 3A-5B illustrate a single convex plate with needles, the eye gripping device may also comprise multiple such plates. For example, multiple convex plates may be radially affixed to a central section of the eye gripping device, with configurations such as two plates on opposite sides, or three, four, or more plates arranged around the central portion. These plates may be symmetrically positioned with respect to a central section of the eye gripping device. Moreover, while Figs. 3A-5B illustrate a convex plate with needles, the plate may alternatively be flat. Multiple flat plates with limited surface area may be arranged together to lie substantially along a curved plane, enabling the plates to engage the curvature of the eyeball when the eye gripping device is applied to the eye. This configuration may allow the eye gripping device to maintain proper contact and functionality, even with flat plates, by positioning and orienting the flat plates collectively to conform to the shape of the eyeball.

In general, the placement of the needles on the front of the eye may be in two primary regions: the sclera, including the thin layer of conjunctiva covering the sclera, and the cornea. It may be desirable to keep the corneal surface largely clear to allow for a line of sight for a microscope. Thus, preferably, only the edge of the cornea is utilized for needle placement. Since the sclera is weaker than the cornea, stresses may need to be distributed more broadly across the sclera. However, the sclera provides a larger surface area for needle placement, allowing for a greater number of needles to be used, which helps in distributing the stresses more effectively. Additionally, utilizing a larger surface area on the sclera may create a stiffer connection closer to the area where instruments are applied to the eye. A combination of these locations may also be employed for optimal gripping performance.

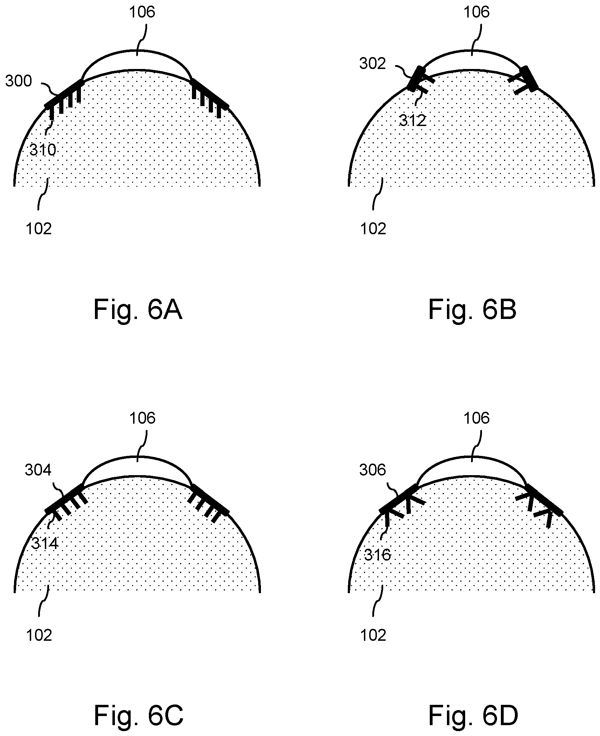

In general, the force required to insert but also to remove the needles is likely lowest in the direction of insertion, which aligns with the length of the needle. To achieve a stronger connection, the needles may be inserted in various directions. Examples of different needle orientations are illustrated in Figs. 6A-6D.

**Fig. 6A** depicts a configuration where the needles 310 are oriented vertically, e.g., in a parallel manner, rather than orthogonal to the ocular surface. In this configuration, the contact element 300 may provide grip against rotation in the direction tangential to the eyeball surface. This configuration may require an axial preload with respect to the needle orientation to maintain the needles' position within the eye.

**Fig. 6B** shows a configuration where the needles 312 grip the cornea. The cornea, being a tougher and harder part of the eye, may allow for a firm grip. The needles 312 may be inserted radially into the cornea near the corneal limbus, providing a secure grip on the eye. This configuration may allow for eye fixation and manipulation in all degrees of freedom (DoF) of the eye gripping device.

**Fig. 6C** shows a configuration where the needles 314 grip the most anterior section of the sclera, adjacent to the corneal limbus. In this region, the conjunctiva may be thinnest. By piercing through the conjunctiva where the conjunctiva loosely slides over the sclera, the needles 314 may grip into the tougher underlying sclera. When the needles are oriented radially to the eyeball surface, this configuration may permit eye fixation and manipulation in all DoF of the eye gripping device.

**Fig. 6D** presents an alternative configuration where the needles 316 are inserted in different directions, such as in a V-shape configuration. This configuration, as shown in conjunction with contact element 306, may allow for an even firmer grip on the eye, further enhancing the stability and control provided by the eye gripping device.

In general, the depth of the needles penetrating into the tissue may be shallow, not extending completely through the fibrous surface layer. The outer diameter of the needles may be small, for example, 29G (approximately 320 µm) or less, preferably 33G (approximately 200 µm) or less, and more preferably 36G (approximately 160 µm) or less. The needles may be positioned orthogonally with respect to the sclera or may be inserted radially into the cornea near the corneal limbus. Alternatively, the needles may be placed in the most anterior section of the sclera, adjacent to the corneal limbus, in a radial orientation. Additionally, as shown previously in Figs. 6A-6D, the microneedles may have varying orientations relative to the fibrous surface layer. For example, groups of microneedles may be arranged in opposing directions with respect to the surface normal of the eye, for example forming a V-shaped configuration. A varied orientation may enhance the grip and stability of the eye gripping device by reducing the likelihood of a common weak point in the attachment.

In general, the surface with which the eye gripping device engages the eye may have various shapes, such as a ring-shaped configuration. Alternatively, the engaging surface (elsewhere also referred to as `contact surface') may be composed of one or more arcuate segments that do not form a complete ring, which may allow more tolerance when engaging with varying eye shapes. Another possible configuration is that the engaging surface may comprise one or more discrete surface patches. An example of such a surface patch is a convex or flat plate, as described elsewhere in this specification. These different configurations allow for flexibility in how the device conforms to and interacts with the eye, providing options for optimized contact and grip depending on the specific application and design requirements.

With continued reference to the eye manipulation system of Figs. 3A-3B, it is noted that the arm may generally have a number of degrees of freedom which may allow the eye gripping device to be moved along the curved plane representing the surface of the eyeball. In another example, the arm may have one degree of movement, for example only along the frontal axis in the nasal-temporal direction or only along the longitudinal axis in the superior-inferior direction. In some examples, the eye manipulation system may comprise an actuation subsystem for actuating the arm and a processor subsystem configured to control the actuation subsystem to move the eye gripping device along the curved plane. For example, the actuation subsystem may comprise one or more actuators, and the processor subsystem may control the actuators accordingly. In a specific example, the actuators may comprise servo motors, stepper motors, or pneumatic cylinders, which provide controlled movement. The actuators may be controlled by the processor subsystem using various methods such as pulse-width modulation (PWM), digital signals, or analog control voltages. The processor subsystem may use feedback mechanisms such as encoders, potentiometers, or linear variable differential transformers (LVDTs) to monitor and adjust the position and speed of the actuators. Optionally, the processor subsystem may implement control strategies such as PID (Proportional-Integral-Derivative) control, feedforward control, or adaptive control.

In some examples, the arm may have multiple degrees of freedom, for example to allow the eye gripping device to be freely moved in a 3D volume within 3D physical space. The degrees of freedom of the arm may be aligned with axes of a coordinate system, for example a 3D Cartesian or a 3D polar coordinate system. In such examples, the processor subsystem may be configured to control the pose of the arm in the coordinate system to cause the eye gripping device to move along the curved plane. For control purposes, the processor subsystem may use a geometric model to internally represent the curved plane. For example, the geometric model may be a geometric model of the eye, for example a model of a surface or outline of the eye. In a specific example, the model of the eye may be a spherical model or an ellipsoid model to internally represent the curved plane. In another specific example, the model may be a patient-specific model, for example a parameterized spherical model or a parameterized ellipsoid model of which the parameters may have been previously fitted to measurement points indicative of an actual geometry of the eye of a patient. For example, a sphere may be parameterized by its center point (*x*₀, *y*₀, *z*₀) and its radius r, while an ellipsoid may be parameterized by its center point (*x*₀, *y*₀, *z*₀), semi-axes (*r*₁, *r*₂, *r*₃) and orientation (*α*, *β*, *γ*)*.* Techniques such as least squares fitting, optimization algorithms, or Hough transforms may be used to derive the parameters of a respective model from a set of measured surface points or from imaging data.

Alternatively to the arm having multiple degrees of freedom to allow the eye gripping device to be freely moved in a 3D volume within 3D physical space, the arm may be designed to mechanically limit movement of the eye gripping device to the curved plane. For example, the arm may be pivoting arm which may enable the eye gripping device to be moved along the curved plane while also limiting the movement of the eye gripping device beyond the curved plane. The pivoting arm may have length of the approximate radius of the eye. For example, the length may be approximately 12mm. The length of the pivoting arm may also be selected to correspond to the length of one of the semi-axes of the eye, e.g., a minor semi-axis or a major semi-axis.

Alternatively, or in addition to being actuated by one or more actuators, the arm may be manually actuatable. This may allow the user to move the eye gripping device along the curved plane by manually adjusting and thereby actuating the arm. For example, when the arm is mechanically designed to limit movement of the eye gripping device to the curved plane, such actuation may be easily effected by the user through simple physical actions such as pushing, pulling, or rotating of an arm part.

In some examples, the eye manipulation system as described in the present disclosure may be combined with a surgical robotic system. For example, both systems may be mechanically aligned or connected, and may optionally share a processor subsystem which may jointly control respective actuation subsystems of the surgical robotic system and the eye manipulation system. In a specific example, the eye manipulation system may be part of the surgical robotic system, for example a subsystem thereof. In yet other examples, the eye manipulation system may be a standalone system or may be part of another system.

Although not shown in the figures, the eye manipulation system may further comprise a sensor configured to quantify the grip of the eye gripping device on the fibrous surface layer of the eyeball, for example to determine an efficacy of the grip or to determine a force exerted on the eye. Examples of such sensors include, but are not limited to, strain gauges, force sensors, piezoelectric sensors, or optical sensors. The processor subsystem of the eye manipulation system may analyze the sensor data of the sensor to quantify the grip, and based on said quantification, take actions such as adjusting the grip of the eye gripping device, aborting a surgical procedure, limiting the movement of the eye gripping device, disconnecting the eye gripping device from the arm etc.

The eye manipulation system may further comprise a sensor data interface to access intraoperative pressure data acquired by an intraoperative pressure sensor. The eye manipulation system may be configured to use the intraoperative pressure data to verify that the eye manipulation system is operating within safe limits. For example, when the intraoperative pressure data indicates that the intraoperative pressure exceeds a safety threshold, the release mechanism of the eye manipulation system may be activated so that the grip exerted on the eye may be released.

It should be noted that the above-mentioned embodiments illustrate rather than limit the presently disclosed subject matter, and that those skilled in the art will be able to design many alternative embodiments without departing from the scope of the appended claims.

In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The mere fact that certain measures are recited in mutually different dependent claims does not indicate that a combination of these measures cannot be used to advantage.

## Claims

1. An eye gripping device (120) for engaging an eyeball, comprising:
- a base structure comprising one or more contact elements (126, 200, 202, 310-316), wherein the one or more contact elements define one or more contact surfaces for contacting an ocular surface of the eyeball, wherein the one or more contact surfaces are arranged along a contact plane (210, 212) that conforms to a curvature of the ocular surface;
- a plurality of needles (128, 230, 310-316) affixed to the base structure, wherein respective needles have an outer diameter of 600 µm or less, preferably 400 µm or less, more preferably 200 µm or less, and protrude by 600 µm or less, preferably 400 µm or less, from the contact plane.

2. The eye gripping device (120) according to claim 1, wherein the base structure comprises a central section (122) which comprises an aperture (124) to expose at least part of the cornea when the eye gripping device engages the eyeball.

3. The eye gripping device (120) according to claim 2, wherein the one or more contact elements (126) are arranged radially around the central section (122) to contact respective parts of the sclera when the eye gripping device engages the eyeball.

4. The eye gripping device (120) according to claim 3, wherein the one or more contact elements (126) form a ring-shaped structure or one or more of radial segments of a ring-shaped structure, wherein the ring-shaped structure has an inner diameter of at least 8 mm, preferably at least 10 mm, more preferably 12 mm.

5. The eye gripping device (120) according to any one of claims 1 to 4, wherein the plurality of needles (128, 230, 310-316) protrude from the one or more contact surfaces of the one or more contact elements.

6. The eye gripping device (120) according to claim 5, wherein the plurality of needles (128, 230, 310-316) are arranged as respective needle beds on the one or more contact surfaces.

7. The eye gripping device (120) according to any one of claims 1 to 6, wherein a protrusion depth (220, 222) of a respective needle is selected to remain below an average physiological thickness of the sclera at a respective position of the needle.

8. The eye gripping device (120) according to any one of claims 1 to 7, wherein the plurality of needles (230) protrude by different lengths from the contact plane (210, 212), wherein needles which are affixed more centrally to the base structure protrude less from the contact plane than needles which are affixed more peripherally to the base structure.

9. The eye gripping device (120) according to any one of claims 1 to 8, further comprising an adjustment mechanism to adjust a protrusion depth of the plurality of needles (230).

10. The eye gripping device (120) according to claim 9, wherein the plurality of needles (230) is slidingly affixed to the base structure, wherein the adjustment mechanism comprises a driving element (240) arranged to drive the needles from the base structure to further protrude from an eye-facing side of the base structure.

11. The eye gripping device (120) according to claim 10, wherein the base structure comprises a threaded portion and the driving element (240) is configured to be screwed onto the threaded portion, wherein the driving element is configured to contact proximal ends of respective needles (230) to drive the needles from the base structure when the driving element is screwed onto the threaded portion.

12. The eye gripping device (120) according to any one of claims 9 to 11, wherein the adjustment mechanism is arranged to sequentially adjust the protrusion depth of individual needles or individual subsets of needles (230).

13. The eye gripping device (120) according to claim 12, wherein the adjustment mechanism is arranged to sequentially adjust the protrusion depth of the individual needles or individual subset of needles (230) by sequentially driving the individual needles or individual subset of needles with the driving element (240) from the base structure.

14. The eye gripping device (120) according to any one of claims 1 to 13, wherein respective needles (128, 230, 310-316) comprise a beveled tip.

15. An eye manipulation system (150) for orbital manipulation of an eye of a patient, wherein the eye manipulation system comprises the eye gripping device (120) according to any one of claims 1 to 14.
